# EUROPEAN PATENT APPLICATION

(11) **EP 4 700 040 A1**
(43) Date of publication of application: **25.02.2026**
(21) Application number: 24792160.4
(22) Date of filing: 19.04.2024
(51) Int. Cl.: C07K 7/06, G01N 33/542, G01N 21/64, G01N 33/53, G01N 33/68, G01N 21/76, C07K 14/435, C07K 19/00, C12N 15/12, C12N 15/62, C12Q 1/66

(54) **PEPTIDE AND LUMINESCENT COMPLEX CONTAINING SAME**

(30) Priority: 21.04.2023 CN 202310442855
(71) Applicant: Shenzhen Loggene Life Science & Technology Co., Ltd., Shenzhen, Guangdong 518000 (CN)
(72) Inventor: ZHANG, Jianliang, Shenzhen, Guangdong 518000 (CN); YANG, Zheng, Shenzhen, Guangdong 518000 (CN); YANG, Lixiang, Shenzhen, Guangdong 518000 (CN); XIAO, Rong, Shenzhen, Guangdong 518000 (CN)
(74) Representative: Calysta NV
(86) International application number: PCT/CN2024/088964
(87) International publication number: WO 2024/217577

(57) **Abstract**

Provided are a series of novel complementary luciferase small subunits, a luminescent complex formed by the association of the complementary luciferase small subunits with complementary luciferase large subunits, and a detection reagent. The obtained small subunits and large subunits are complementary to each other to detect a target, such that vitality, a signal-to-noise ratio, senitivity, and a linear range are significantly improved, thereby making it possible to use the novel complementary luciferase small subunits for clinical sample diagnosis.

## Description

### FIELD OF THE INVENTION

The present invention relates to the field of luciferase-catalyzed luminescent reaction, and specifically to a series of novel complementary luciferase small subunit peptides and a luminescent complex comprising the same.

### BACKGROUND OF THE INVENTION

Luciferase refers to a general term for enzymes that can produce bioluminescence in nature. Luciferase can catalyze the reaction in which luciferin is oxidized into oxyluciferin, and the luciferin can emit bioluminescence as it is oxidized. The bioluminescence released during the oxidation of luciferin can then be determined by instruments. Luciferase usually encounters severe matrix interference from body fluid samples in the diagnosis, especially from albumin in blood samples. The activity of luciferase decreases significantly after the addition of blood samples, and the degree of decrease in activity varies from sample to sample, which seriously affects the application of this enzyme in clinical diagnosis.

A two-subunit system based on a novel luciferase divides the luciferase into two subunits, a large subunit and a small subunit. When the two subunits combined, an active and complementary luciferase is obtained, which can thus react with the substrate to produce luminescence for detecting molecular interactions between target molecules. It has been found that the existing complementary luciferases have too low activity in large and/or small subunits and high detection background when detecting the target, making it difficult to obtain high sensitivity with a wide linear range.

Therefore, there is a need to develop novel complementary fragments of luciferase with the aim of having a higher signal-to-noise ratio in complementary activation of the detection target, thereby enhancing the sensitivity of the detection.

### SUMMARY OF THE INVENTION

The present invention provides a series of complementary luciferase small subunit peptides with a high signal-to-noise ratio. When complemented with a large subunit to detect the target, they significantly improve in terms of signal-to-noise ratio, activity, sensitivity and linear range, making their use in the diagnosis of clinical samples possible.

In a first aspect, the present invention provides a peptide, the peptide comprising an amino acid sequence of 11 adjacent amino acids, X₁X₂X₃X₄X₅X₆X₇X₈X₉X₁₀X₁₁, wherein X₁ is Val, X₂ is selected from Glu, Ser, Asp, or Asn, X₃ is selected from Gly, X₄ is selected from Tyr or Trp, X₅ is selected from Arg, X₆ is selected from Leu or Ile, X₇ is selected from Phe or His, X₈ is selected from Asp, Glu, Asn, or Lys, X₉ is selected from Lys, Arg, or Glu, X₁₀ is selected from Lys, Val, Ile, or Glu, and X₁₁ is selected from Ser, Trp, Leu, or Gly.

In some embodiments, in the amino acid sequence, X₁ is Val, X₂ is selected from Glu, Ser, Asp, or Asn, X₃ is selected from Gly, X₄ is selected from Tyr or Trp, X₅ is selected from Arg, X₆ is selected from Leu or Ile, X₇ is selected from Phe or His, X₈ is selected from Asp, Glu, Asn, or Lys, X₉ is selected from Lys, Arg, or Glu, X₁₀ is selected from Lys, Val, or Glu, and X₁₁ is selected from Ser, Trp, Leu, or Gly.

In some preferred embodiments, in the amino acid sequence, X₁ is Val, X₂ is selected from Glu or Asp, X₃ is selected from Gly, X₄ is selected from Tyr or Trp, X₅ is selected from Arg, X₆ is selected from Leu or Ile, X₇ is selected from Phe or His, X₈ is selected from Asp, Glu, Asn, or Lys, X₉ is selected from Lys, Arg, or Glu, X₁₀ is selected from Lys, Val, Ile, or Glu, and X₁₁ is selected from Ser, Trp, or Leu.

In the present application, the peptide having the specific amino acid sequence described above is a small subunit of a complementary luciferase. When it is associated with a wild-type large subunit of the complementary luciferase, or a truncation or mutant thereof, a detectable luminescent signal is produced in the presence of a substrate. In some embodiments, the substrate comprises coelenterazine or an analog thereof.

In some embodiments, when the peptide contacts a second peptide having an amino acid sequence with at least 70%, 75%, 80%, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or 99.9% sequence identity to SEQ ID NO: 86, a detectable luminescent signal is produced in the presence of a substrate.

In some embodiments, the second peptide has a sequence shown in SEQ ID NO: 86:

VFTLEDFVGDWEQTAAYNLDQVLEQGGVSSLLQNLAVSVTPIQRIVRSGENALKID
IHVIIPYEGLSADQMAQIEEVFKVVYPVDDHFKVILPYGTLVIDGVTPNMLNYFGRPYE
GIAVFDGKKKITVTGTLWNGNKIIDERLITPDGSMLFRVTINS (SEQ ID NO: 86).

In some embodiments, the peptide is associated with the second peptide.

In some embodiments, the peptide has an amino acid sequence selected from at least one of SEQ ID NOs: 1-31 and SEQ ID NOs: 33-74:
VEGYRLFDKKW (SEQ ID NO: 1), VDGWRLHEKIS (SEQ ID NO: 2),
VEGYRLFDRKS (SEQ ID NO: 3), VEGWRLHEKIS (SEQ ID NO: 4),
VEGYRLHEKIS (SEQ ID NO: 5), VEGYRLFDRKW (SEQ ID NO: 6),
VEGYRLFDRKL (SEQ ID NO: 7), VEGYRLFKREL (SEQ ID NO: 8),
VEGYRLFDRVW (SEQ ID NO: 9), VEGYRLFEKVW (SEQ ID NO: 10),
VSGWRIHEKIS (SEQ ID NO: 11), VEGYRLFEKVW (SEQ ID NO: 12),
VEGYRLFERIW (SEQ ID NO: 13), VEGYRLFERKW (SEQ ID NO: 14),
VEGYRLFNRKW (SEQ ID NO: 15), VEGYRLFDRKL (SEQ ID NO: 16),
VEGYRLFDRKL (SEQ ID NO: 17), VEGYRLFERVL (SEQ ID NO: 18),
VEGYRLFERKW (SEQ ID NO: 19), VEGYRLFERIW (SEQ ID NO: 20),
VEGYRLFNEKS (SEQ ID NO: 21), VEGYRLFNRKS (SEQ ID NO: 22),
VEGYRLFERKS (SEQ ID NO: 23), VEGYRLFNKKS (SEQ ID NO: 24),
VEGYRLFERKL (SEQ ID NO: 25), VEGYRLFNRVS (SEQ ID NO: 26),
VEGYRLFERKS (SEQ ID NO: 27), VEGYRLFEKKL (SEQ ID NO: 28),
VEGYRLFNRKS (SEQ ID NO: 29), VEGYRLFNRVG (SEQ ID NO: 30),
VEGYRLFNKEW (SEQ ID NO: 31), VDGWRLFDKKS (SEQ ID NO: 33),
VEGYRIFEKKW (SEQ ID NO: 34), VEGYRLFDKVS (SEQ ID NO: 35),
VSGYRLFDRKW (SEQ ID NO: 36), VEGYRLFERKS (SEQ ID NO: 37),
VEGYRLFEKVS (SEQ ID NO: 38), VSGYRLFDKVS (SEQ ID NO: 39),
VDGWRLFDKKS (SEQ ID NO: 40), VEGYRLFDKVS (SEQ ID NO: 41),
VEGYRLFDKVS (SEQ ID NO: 42), VSGYRIFDKKS (SEQ ID NO: 43),
VSGYRLFDKKS (SEQ ID NO: 44), VEGYRLFDRKS (SEQ ID NO: 45),
VSGYRLFDKKS (SEQ ID NO: 46), VDGWRLFERKS (SEQ ID NO: 47),
VSGWRIFDKKS (SEQ ID NO: 48), VSGYRLFDKKW (SEQ ID NO: 49),
VDGYRLFEKVS (SEQ ID NO: 50), VSGYRLFDRKS (SEQ ID NO: 51),
VDGYRLFERKS (SEQ ID NO: 52), VSGYRLFDKVS (SEQ ID NO: 53),
VDGWRLFDKKS (SEQ ID NO: 54), VSGYRLFDKVS (SEQ ID NO: 55),
VEGYRLFEKIS (SEQ ID NO: 56), VNGWRIFDKKS (SEQ ID NO: 57),
VSGYRIFERKW (SEQ ID NO: 58), VSGYRIFEKKS (SEQ ID NO: 59),
VEGYRLFEKIS (SEQ ID NO: 60), VEGWRLFDRKS (SEQ ID NO: 61),
VSGYRLFDRVW (SEQ ID NO: 62), VSGYRIFEKKS (SEQ ID NO: 63),
VEGYRLFEKIS (SEQ ID NO: 64), VSGYRLFDRKS (SEQ ID NO: 65),
VSGYRIFEKKW (SEQ ID NO: 66), VEGWRIFERKW (SEQ ID NO: 67),
VSGYRLFDRKS (SEQ ID NO: 68), VNGWRLFDKKS (SEQ ID NO: 69),
VEGYRLFERKS (SEQ ID NO: 70), VEGWRLFDRVS (SEQ ID NO: 71),
VSGYRLFDRVS (SEQ ID NO: 72), VSGWRIFDRKS (SEQ ID NO: 73), and
VSGYRLFDKKW (SEQ ID NO: 74).

In some embodiments, the peptide has a signal-to-noise ratio of ≥ 12, preferably ≥ 27, more preferably ≥ 30 for luciferase dual-antibody complementary target detection, when a concentration of the peptide is 30-360 nM, a concentration of the second peptide is 15-200 nM (e.g., 16.67-200 nM), and a concentration of the target to be detected is 1-120 pM (e.g., 24.4-118 pM). Herein, the concentration of the target to be detected is the final concentration in the substrate-free incubation system.

In some embodiments, the peptide has a signal-to-noise ratio of ≥ 2, preferably ≥ 5, more preferably ≥ 7 for luciferase dual-antibody complementary target detection, when a concentration of the peptide is 0.2-30 nM (e.g., 0.234-30 nM), a concentration of the second peptide is 0.2-20 nM (e.g., 0.234-16.67 nM), and a concentration of the target to be detected is 1-100 pM (e.g., 19.5-93 pM). Herein the concentration of the target to be detected is the final concentration in the substrate-free incubation system.

In a second aspect, the present invention provides a nucleic acid molecule, comprising a nucleotide sequence encoding a peptide described in the first aspect.

In a third aspect, the present invention provides an expression vector, comprising a nucleotide sequence of a nucleic acid molecule described in the second aspect.

In a fourth aspect, the present invention provides a host cell, comprising an expression vector described in the third aspect.

In a fifth aspect, the present invention provides a conjugate, which as a fusion polypeptide comprises a first domain and a second domain, wherein the first domain comprises the peptide described in the first aspect; and the second domain comprises a first ligand.

In some embodiments, the first ligand is selected from an antigen or antigen-binding region.

In some embodiments, the antigen-binding region comprises antibody Fab fragment, Fab' fragment, F(ab')₂ fragment, Fv fragment, scFv fragment, nanobody, heavy chain variable region (VH) fragment, or light chain variable region (VL) fragment. In some embodiments, the first antigen-binding region comprises a scFv fragment.

In some embodiments, the antigen includes, but is not limited to: procalcitonin, interleukin 6, and the like.

In some embodiments, the first domain and the second domain are connected directly or via a first linker. In some embodiments, the first linker is selected from a polypeptide linker and/or a non-polypeptide linker.

In some embodiments, the first domain and the second domain form a fusion polypeptide via a polypeptide linker.

In some embodiments, the first linker is a flexible linker having a formula of (G)ₙS(G)ₘS(G)ₗS, wherein n is selected from 1 to 5, m is selected from 0 to 5, and l is selected from 0 to 5.

In some embodiments, the first linker is a flexible linker having a formula of (G)ₙSS(G)ₗS, wherein n is selected from 1 to 5, and l is selected from 0 to 5. In some embodiments, the first linker is a flexible linker having a formula of (G)ₙS(G)ₘSS, wherein n is selected from 1 to 5, and m is selected from 0 to 5. In some embodiments, the first linker is a flexible linker having a formula of (G)ₙSSS, wherein n is selected from 1 to 5.

In some embodiments, the first linker comprises GGGS, GGGSGGS (SEQ ID NO: 93), GGGSGGGGS (SEQ ID NO: 94), GGGSGGGSGGGS (SEQ ID NO: 95), or GGGGSGGGGSGGGGS (SEQ ID NO: 96).

In some embodiments, the first linker is a rigid linker having a formula of A(EAAAK)ₒA, wherein o is selected from 0 to 5.

In some embodiments, the first linker comprises AEAAAKA (SEQ ID NO: 97), AEAAAKEAAAKA (SEQ ID NO: 98), or AEAAAKEAAAKEAAAKA (SEQ ID NO: 99).

In some embodiments, the first linker is a chemical linker.

In some embodiments, the first domain and the second domain are covalently connected via a chemical linker to form a coupled polypeptide.

In some embodiments, the first linker comprises one or more of acrylic phosphoramidite, adenylation, azide, digoxigenin, cholesterol-TEG, amino modifier, alkyne, biotinylation, thiol modification, tetraethylene glycol (TEG), hexaethylene glycol (HEG), polyethylene glycol (PEG), glycerol, C2 to C12 alkyl and succinimide.

In some embodiments, the first domain and the second domain are connected via a polypeptide linker and a chemical linker. In some specific embodiments, the first domain and the second domain are connected via a GSlinker and an SMCC coupling agent, wherein one end of the SMCC coupling agent is coupled to a cysteine at the terminus of the first domain, and the other end is coupled to a primary amino group of the second domain.

In some embodiments, the first domain and the second domain are directly connected via 1-ethyl-(3-dimethylaminopropyl)carbodiimide (EDC).

In a sixth aspect, the present invention provides a nucleic acid molecule, comprising a nucleotide sequence encoding a conjugate described in the fifth aspect.

In a seventh aspect, the present invention provides an expression vector, comprising a nucleotide sequence of a nucleic acid molecule described in the sixth aspect.

In an eighth aspect, the present invention provides a host cell comprising an expression vector described in the seventh aspect.

In a ninth aspect, the present invention provides a luminescent complex comprising:
(a) a conjugate described in the fifth aspect; and
(b) a second conjugate comprising a third domain and a fourth domain, wherein the third domain comprises a second peptide, the fourth domain comprises a second ligand, the second peptide is a structural complementary peptide to the first peptide, and when the first peptide is associated with the second peptide, a detectable luminescent signal is produced in the presence of a substrate.

In some embodiments, the second ligand is selected from an antigen or antigen-binding region.

In some embodiments, the second antigen-binding region comprises antibody Fab fragment, Fab' fragment, F(ab')₂ fragment, Fv fragment, scFv fragment, nanobody, heavy chain variable region (VH) fragment or light chain variable region (VL) fragment. In some embodiments, the second antigen-binding region comprises a scFv fragment.

In some embodiments, the third domain and the fourth domain are connected directly or via a second linker.

In some embodiments, the second linker is selected from a polypeptide linker and/or a non-polypeptide linker.

In some embodiments, the third domain and the fourth domain form a fusion polypeptide via a polypeptide linker.

In some embodiments, the second linker is a flexible linker peptide having a formula of GSS(G)ₚ(S)_{q}(G)ᵣ(S)ₛ(G)ₜ(S)ᵤ, wherein p is selected from 1 to 5, q is selected from 0 to 5, r is selected from 0 to 5, s is selected from 0 to 1, t is selected from 0 to 5, and u is selected from 0 to 5.

In some embodiments, the second linker is a flexible linker having a formula of GSS(G)ₚSS(G)ₜS, wherein p is selected from 1 to 5, and t is selected from 0 to 5. In some embodiments, the second linker is a flexible linker having a formula of GSS(G)ₚS(G)ᵣSS, wherein p is selected from 1 to 5, and r is selected from 0 to 5. In some embodiments, the second linker is a flexible linker having a formula of GSS(G)ₚSSS, wherein p is selected from 1 to 5.

In some embodiments, the second linker comprises a sequence selected from the group consisting of GSSG, GSSGGGS (SEQ ID NO: 100), GSSGGGSGGS (SEQ ID NO: 101), GSSGGGGSGGGS (SEQ ID NO: 102) or GSSGGGGSGGGGSGG (SEQ ID NO: 103).

In some embodiments, the second linker is a chemical linker.

In some embodiments, the third domain and the fourth domain are covalently connected via a chemical linker to form a coupled polypeptide.

In some embodiments, the second linker comprises one or more of acrylic phosphoramidite, adenylation, azide, digoxigenin, cholesterol-TEG, amino modifier, alkyne, biotinylation, thiol modification, tetraethylene glycol (TEG), hexaethylene glycol (HEG), polyethylene glycol (PEG), glycerol, C2 to C12 alkyl and succinimide.

In some embodiments, the first domain and the second domain are connected via a polypeptide linker and a chemical linker. In some specific embodiments, the first domain and the second domain are connected via a GS linker and an SMCC coupling agent, wherein one end of the SMCC coupling agent is coupled to a cysteine at the terminus of the first domain, and the other end is coupled to a primary amino group of the second domain.

In some embodiments, the third domain and the fourth domain are directly connected via 1-ethyl-(3-dimethylaminopropyl)carbodiimide (EDC).

In the present application, the first peptide is a small subunit of a complementary luciferase, and the second peptide is a wild-type large subunit of a complementary luciferase, or a truncation or mutant thereof. When the first peptide of the present application is associated with the second peptide, a detectable luminescent signal is produced in the presence of a substrate. In some embodiments, the substrate comprises coelenterazine or an analog thereof.

In some embodiments, the second peptide comprises an amino acid sequence with at least 70%, 75%, 80%, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or 99.9% sequence identity to SEQ ID NO: 86.

In some embodiments, the second peptide has a sequence shown in SEQ ID NO: 86:

VFTLEDFVGDWEQTAAYNLDQVLEQGGVSSLLQNLAVSVTPIQRIVRSGENALKID
IHVIIPYEGLSADQMAQIEEVFKVVYPVDDHFKVILPYGTLVIDGVTPNMLNYFGRPYE
GIAVFDGKKKITVTGTLWNGNKIIDERLITPDGSMLFRVTINS (SEQ ID NO: 86).

In some embodiments, the conjugate described in the fifth aspect of the present invention is associated with the second conjugate.

In a tenth aspect, the present invention provides another luminescent complex, comprising:(a) a first peptide comprising the peptide described in the first aspect; and (b) a second peptide being a structurally complementary peptide to the first peptide, wherein when the first peptide is associated with the second peptide, a detectable luminescent signal is produced in the presence of a substrate.

In some embodiments, the second peptide comprises an amino acid sequence with at least 70%, 75%, 80%, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or 99.9% sequence identity to SEQ ID NO: 86.

In some embodiments, the second peptide has a sequence shown in SEQ ID NO: 86:

VFTLEDFVGDWEQTAAYNLDQVLEQGGVSSLLQNLAVSVTPIQRIVRSGENALKID
IHVIIPYEGLSADQMAQIEEVFKVVYPVDDHFKVILPYGTLVIDGVTPNMLNYFGRPYE
GIAVFDGKKKITVTGTLWNGNKIIDERLITPDGSMLFRVTINS (SEQ ID NO: 86).

In an eleventh aspect, the present invention provides a detection reagent, comprising: (a) a first peptide comprising a peptide described in the first aspect; and (b) a substrate for a luminescent complex formed by association of the first peptide and a second peptide; wherein the second peptide is a structurally complementary peptide to the first peptide, and when the first peptide is associated with the second peptide, a detectable luminescent signal is produced in the presence of the substrate.

In some embodiments, the second peptide comprises an amino acid sequence with at least 70%, 75%, 80%, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or 99.9% sequence identity to SEQ ID NO: 86.

In some embodiments, the second peptide has a sequence shown in SEQ ID NO: 86:

VFTLEDFVGDWEQTAAYNLDQVLEQGGVSSLLQNLAVSVTPIQRIVRSGENALKID
IHVIIPYEGLSADQMAQIEEVFKVVYPVDDHFKVILPYGTLVIDGVTPNMLNYFGRPYE
GIAVFDGKKKITVTGTLWNGNKIIDERLITPDGSMLFRVTINS (SEQ ID NO: 86).

In a twelfth aspect, the present invention provides use of a peptide described in the first aspect, a conjugate described in the fifth aspect, a nucleic acid described in the second aspect or the sixth aspect, an expression vector described in the third aspect or the seventh aspect, a host cell described in the fourth aspect or the eighth aspect, a luminescent complex described in the ninth aspect or the tenth aspect, or a detection reagent described in the eleventh aspect, in a platform based on luciferase luminescence detection.

In a thirteenth aspect, the present invention also provides a method of detection, comprising the steps of:
(1) forming a first fusion protein from the peptide described in the first aspect and a first antigen-binding region of the antigen to be detected;
(2) forming a second fusion protein from a structurally complementary peptide of the peptide and a second antigen-binding region of the antigen to be detected;
(3) incubating a substance to be detected with the first fusion protein and the second fusion protein; preferably incubating for 3-10 min at between 35°C and 40°C;
(4) adding a substrate for incubation; preferably, the substrate is coelenterazine and/or an analog thereof, e.g., Furimazine or CTZ-h; and
(5) detecting the luminescence signal by a bioluminescence detector.

The beneficial effects of the present invention compared to the prior art are:
The luciferase small subunits obtained in the present invention, when complemented with large subunit for detection targets, have higher luminescent signal value, lower background, higher detection sensitivity and wider linear range; thus, making it possible to apply complementary luciferase technology to the diagnosis of clinical samples.

### BRIEF DESCRIPTION OF THE DRAWINGS

Figure 1 illustrates the schematic diagram of the principle of the complementary luciferase double antibody (SCFV) for complementary target detection.
Figure 2 illustrates a standard curve of the complementary activation of different small subunit mutant-rSCFV18 and large subunit-rSCFV5 fusion proteins for PCT detection.
Figure 3 illustrates a standard curve of the complementary activation of different small subunit mutant-rSCFV18 and large subunit-rSCFV5 fusion proteins for PCT detection.

### DETAILED DESCRIPTION OF THE EMBODIMENTS

In order to make the objects, technical solutions and advantages of the present invention clearer and more understandable, the present invention is described in further detail in the following in conjunction with examples. The specific examples described herein are for the sole purpose of explaining the present invention and are not intended to constitute any limitation of the present invention. In addition, in the following description, descriptions of well-known structures and techniques are omitted to avoid unnecessary confusion about the concepts of the present disclosure. Such structures and techniques are also described in many publications.

### Definition

Unless otherwise defined, all technical and technological terms used in the present invention have the same meaning as commonly used in the field to which the present invention belongs. For the purpose of interpreting this specification, the following definitions will be applied and, where appropriate, terms used in the singular form will also include the plural form and vice versa.

The expressions "a" and "an" as used herein include the plural references unless the context clearly indicates otherwise.

The term "antigen" as used herein refers to a site on a polypeptide macromolecule (e.g., a continuous amino acid sequence or a conformational configuration composed of disparate regions of non-contiguous amino acids). The antigen-binding region binds the site, thereby forming an antigen-binding region-antigen complex. Unless otherwise indicated, the proteins used as antigen in the present invention may be any natural form of protein from any vertebrate source, the vertebrate sources including mammals such as primates (e.g., humans) and rodents (e.g., mice and rats). Antigen may also be a human protein, or antigen may be "full-length", unprocessed protein, and any form of protein resulting from intracellular processing, or a naturally occurring variant of protein, such as a splice variant or allelic variant. Antigen may also be a small molecule compound.

The term "antibody" as used herein includes an intact antibody and any antigen-binding fragment (i.e., "antigen-binding portion", "antigen-binding region", "antigen-binding polypeptide" or "immunoconjugate"), or single chains thereof. A single chain antibody fragment (scFv) is an antibody consisting of a heavy chain variable region and a light chain variable region of an antibody connected via a short peptide (linker) of 15-20 amino acids. scFv retains its affinity activity for antigen better and is characterized by small molecular weight, high penetration and weak antigenicity.

The term "fusion polypeptide" as used herein refers to two or more proteins or fragments thereof that are colinearly connected via their respective peptide backbones using genetic expression of a polynucleotide encoding the protein or protein synthesis methods. Preferably, the polypeptide or fragments thereof are from different sources. In some embodiments, the fusion polypeptide comprises the peptide and an antibody against the antigen to be detected. The fusion polypeptide or fragments thereof herein may comprise conservative amino acid substitution at one or more amino acid residues, e.g., at essential or non-essential amino acid residues. "Conservative amino acid substitution" is the replacement of an amino acid residue by an amino acid residue with a similar side chain.

The term "coupled polypeptide" as used herein refers to two or more proteins or fragments thereof that are covalently connected via a non-polypeptide linker using a chemical coupling method. Preferably, the polypeptide or fragments thereof are from different sources. In some embodiments, the coupled polypeptide comprises the peptide and an antibody against the antigen to be detected.

"Percentage (%) sequence identity" relative to a reference amino acid sequence refers to the percentage of amino acid residues in a candidate sequence that are identical to amino acid residues in the reference amino acid sequence after alignment of the sequences and (as needed) introduction of gaps to obtain the maximum percentage of sequence identity, without regard to any conservative substitutions as part of the sequence identity. To determine the percentage of amino acid sequence identity, alignment can be performed in various ways within the scope of the field, such as using BLAST, ALIGN, or Megalign (DNASTAR) software. A person skilled in the art may determine appropriate parameters for alignment of sequences, including any algorithms required to achieve maximum alignment over the full length of the sequences being aligned. In some embodiments, "at least 70% sequence identity" includes about 70%, 75%, 80%, 85%, 90%, 95%, 98%, 99%, 100%, or any value therebetween, of sequence identity.

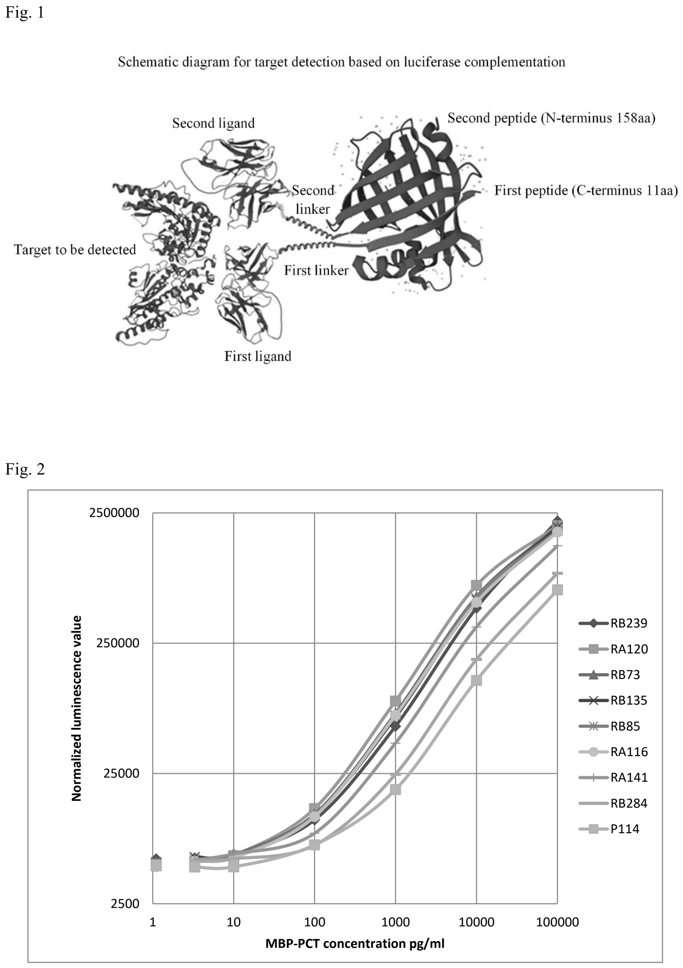

The term "luciferase" as used herein refers to a general term for enzymes that can produce bioluminescence in nature. In the present invention, luciferase is all enzymes that undergo catalytic luminescence in the presence of a substrate such as coelenterazine or an analog thereof, and combinations of enzymes fragments that remain catalytically active after recombination of fragments thereof. The "complementary luciferase" of the present invention is a two-subunit system based on luciferase, comprising two subunits, the large subunit (OL) and the small subunit (OS). The N-terminal large subunit is about 158 amino acids, and the C-terminal small subunit is about 11 amino acids. The two subunits can be expressed as fusion proteins with two target proteins to be detected, respectively. When the interaction between the target proteins causes the two subunits to bind, an active luciferase can be obtained, which can then react with a substrate to produce luminescence for detecting molecular interactions between target molecules (see Figure 1). In some embodiments, the large and small subunits are associated with the target molecule and the bioluminescent complex is operated through molecular interactions of the target molecules. If the target molecules are involved in sufficiently stable interactions, a bioluminescent complex is formed and a bioluminescent signal is produced. If the target molecules are not able to be involved in sufficiently stable interactions, bioluminescent complex cannot be formed or can be formed only weakly and the bioluminescent signal is undetectable or significantly reduced. In some embodiments, the magnitude of the detectable bioluminescent signal is proportional (e.g., directly proportional) to the amount, intensity, facilitation, and/or stability of the molecular interaction between the target molecules.

The term "coelenterazine" as used herein is a luciferin present in seven phyla of aquatic organisms that can be luminescence, and is a substrate for many luciferases and photoproteins. The system consisting of coelenterazine and luciferase using the coelenterazine as a substrate is a common bioluminescence system. In addition to natural coelenterazine, which can be used as a substrate for luciferase, some coelenterazine analogs with different spectroscopic properties, stability and other characteristics are also available. Exemplary coelenterazine analogs include: Coelenterazine h (CTZ-h): (2-deoxycoelenterazine or 2,8-dibenzyl-6-(4-hydroxyphenyl)imidazo[1,2-a]pyrazin-3(7H)-one); Coelenterazine h h (CTZ-h-h): (dideoxycoelenterazine or 2,8-dibenzyl-6-phenylimidazo[1,2-a]pyrazin-3(7H)-one); Furimazine (FZ): (8-benzyl-2-[(furan-2-yl)methyl]-6-phenylimidazo[1,2-a]pyrazin-3(7H)-one); Coelenterazine f (CTZ-f): (8-benzyl-2-(4-fluorophenyl)-6-(4-hydroxyphenyl)imidazo[1,2-a]pyrazin-3(7H)-one); JRW-0238: (8-benzyl-2-(furan-2-yl-methyl)-6-(3-hydroxyphenyl)imidazo[1,2-a]pyrazin-3(7H)-one); JRW-1743: (6-(3-amino-2-fluorophenyl)-8-(2-fluorobenzyl)-2-(furan-2-yl-methyl)imidazo[1,2-α]pyrazin-3(7H)-one); JRW-1744: (6-(3-amino-2-fluorophenyl)-8-benzyl-2-(furan-2-yl-methyl)imidazo[1,2-α]pyrazin-3(7H)-one); other exemplary coelenterazine analogs include coelenterazine n, coelenterazine f, coelenterazine hcp, coelenterazine cp, coelenterazine c, coelenterazine e, coelenterazine fcp, coelenterazine I, coelenterazine icp, coelenterazine v, 2-methylcoelenterazine, etc.

Among them, coelenterazine has the following structure:

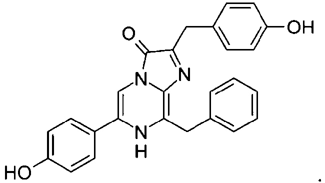

Exemplary coelenterazine analogs have the following structures:

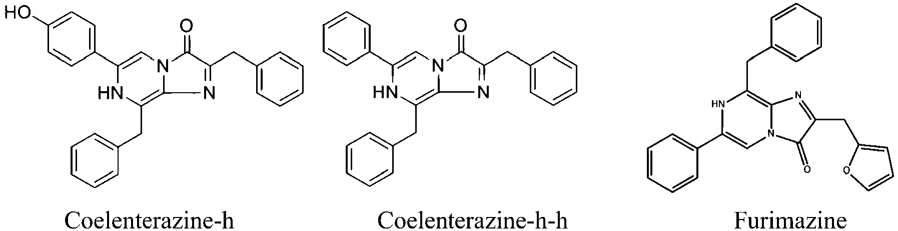

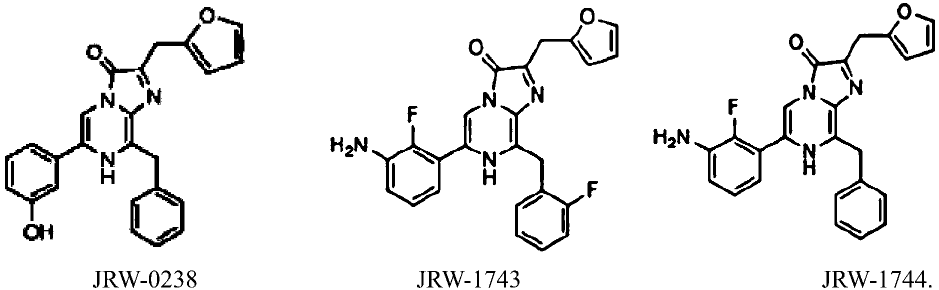

The term "procalcitonin (abbreviated as PCT)" as used herein refers to a protein of an encoding gene located on human chromosome 11. This gene consists of 6 exons and 5 introns, with a total length of 7,600 bp, and a length of coding region is 2,800 bp. PCT is a specific indicator of severe bacterial inflammation and fungal infections, but also a reliable indicator of sepsis and multiple organ failure associated with inflammatory activity. MBP-PCT refers to PCT with maltose-binding protein (MBP) tag.

As used herein, the signal-to-noise ratio refers to the ratio of the luminescence value when a specific amount of fusion polypeptide is used, with a certain amount of the target to be detected, to the luminescence value without the target to be detected. The amounts of the fusion polypeptide and the target can be represented by the concentration when the volume is determined. The signal-to-noise ratio can reflect the sensitivity of the detection. Different addition amounts of target, as well as different addition amounts of fusion polypeptide, can lead to differences in signal-to-noise ratios. Signal-to-noise ratio = luminescence value 1 / luminescence value 2, wherein luminescence value 1 is the luminescence value when a certain amount of target to be detected is added, and luminescence value 2 is the luminescence value when no target to be detected is added.

As used herein, the term "linker" refers to a peptide or polypeptide sequence (e.g., a synthetic peptide or polypeptide sequence) or a non-polypeptide, such as an alkyl chain. In some embodiments, two or more linkers may be connected in series. When multiple linkers exist, each can be the same or different. Generally, the linkers provide flexibility or prevent/improve steric hindrance. The linkers are typically not cleaved; however, in certain aspects, such cleavage may be required. Thus, in some embodiments, the linker may comprise one or more protease cleavable sites that may be located within the linker sequence or the linker flank at either end of the linker sequence.

In some embodiments, the linker is a polypeptide linker. In some embodiments, the peptide linker can comprise at least about two, at least about three, at least about four, at least about five, at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 amino acids. In some embodiments, the linker comprises one or more amino acids.

In some embodiments, the linker is a non-polypeptide linker. In some embodiments, the linker comprises acrylic phosphoramidite (e.g., ACRYDITE^{™}), adenylation, azide (NHS Ester), digoxigenin (NHS Ester), cholesterol-TEG, I-LINKER^{™}, amino modifier (e.g., amino modifier C6, amino modifier C12, amino modifier C6 dT, or Uni- Link^{™} amino modifier), alkyne, 5'-hexynyl, 5-octadiynyl dU, biotinylation (e.g., biotin, biotin (azide), biotin dT, biotin-TEG, dual biotin, PC biotin, or desthiobiotin), thiol modification (thiol modifier C3 S-S, dithiol, or thiol modifier C6 S-S), tetraethylene glycol (TEG), hexaethylene glycol (HEG), polyethylene glycol (PEG), glycerol, C2 to C12 alkyl, succinimide, or any combination thereof.

Examples and drawings are provided below to assist in understanding the present invention. It should be understood, however, that these examples and drawings are intended to illustrate the present invention only, but do not constitute any limitation. The actual scope of protection of the present invention is set forth in the claims. It should be understood that any modifications and changes may be made without departing from the spirit of the present invention.

The reagents used in the following examples are as follows:
(1) PBSA formulation (enzyme diluent):
   0.2 g/L of anhydrous KH₂PO₄, 0.61 g/L of anhydrous Na₂HPO₄, 0.2 g/L of KCl, 8 g/L of NaCl, 1 g/L of BSA, 20 g/L of PEG 20000, 100 µl/L of Triton X-405, 1 ml/L of proclin 300, 1.37 ml/L of ethanol, and pH 7.4.
(2) PBSAD formulation (target diluent):
   0.2 g/L of anhydrous KH₂PO₄, 0.61 g/L of anhydrous Na₂HPO₄, 0.2 g/L of KCl, 8 g/L of NaCl, 1 g/L of BSA, 20 g/L of PEG 20000, 100 µl/L of Triton X-405, 1 ml/L of proclin 300, 1.37 ml/L of ethanol, 1 mM of DTT, and pH 7.4.
(3) Obuffer 6.37-3 formulation (substrate working solution):
   100 mM of MES, 1 mM of CDTA, 0.02% of Tergitol, 150 mM of KCl, 35 mM of Thiorea, 0.10% of proclin 300, and pH 6.0.
(4) Furimazine stock solution formulation:
   10% of methanol, 15% of DMSO, 75% of propylene glycol, and 2.5 mM of Furimazine.
(5) CTZ-h stock solution formulation:
   10% of methanol, 15% of DMSO, 75% of propylene glycol, and 2.5 mM CTZ-h.

The drugs or media used in the following examples are as follows:
Triton X-405, purchased from Aladdin, Cat. No. T101476;
Proclin 300, purchased from Sigma, Cat. No. 48912-V;
DTT (Dithiothreitol), purchased from Sangon Biotech, Cat. No. A620058-0025;
MES (4-morpholine ethanesulfonic acid), purchased from Aladdin, Cat. No. M163013-25g;
CDTA: (1,2 cyclohexane-diamine-tetraacetic acid), purchased from Aladdin, Cat. No. C193548-5g;
Tergitol, purchased from Sigma, Cat. No. 15S9-100ml;
Thiourea, purchased from Aladdin, Cat. No. T112514-500g;
Polymyxin B (polymyxin B sulfate), purchased from Sigma, Cat. No. P1004-25MU;
Furimazine (luminescent substrate for imidazopyrazinones), purchased from Wuhan Chemstan Biotechnology, Cat. No. CS15359;
CTZ-h (coelenterazine h), purchased from Xi'an Biolite Biotech, Cat. No. 21165;
2YTA medium: 10g/L of yeast extract (Thermo, OXOID, LP0021B), 16g/L of Tryptone (Thermo, OXOID, LP0042B), 5g/L of NaCl (Sangon Biotech, A501218-0001). Sterilized by autoclaving. Ampicillin (Sangon Biotech, A610028-0025) is added to a final concentration of 100mg/L before use (after returning to room temperature).

MBP-PCT (procalcitonin (PCT) fused with the maltose-binding protein (MBP) at the N-terminus) was generated by fusing MBP protein to PCT (NP_001029124.1, AA 26-141) via a linker peptide NAEAAAKEAAAKALVPRGSGGGGS (SEQ ID NO: 104). The resulting fusion protein was expressed in E. coli and subsequently affinity purified using MBP protein purification resin. In the following examples, the concentration of MBP-PCT is calculated according to the effective concentration of PCT. For example, a concentration of 1 ng/ml for MBP-PCT means that the effective concentration of PCT in it is 1 ng/ml, whereas the concentration of MBP-PCT that actually needs to be formulated is 1*55.9/12.8/90% = 4.85 ng/ml (the predicted molecular weight of MBP-PCT is 55.9 KD, the predicted molecular weight of PCT is 12.8 KD, and the MBP- PCT's purity was calculated as 90%).

Unless otherwise specified, the other reagents, instruments and the like used in the present invention are commonly used and commercially available in the field and can be purchased in the market.

The present invention designs a series of complementary luciferase small subunit proteins with different sequences, and utilizes a functional screening method to screen for small subunits with lower background and higher detection sensitivity in complementation with the large subunit-antibody for detection; all of the selected small subunits can react well with coelenterazine-like substrates after being associated with the large subunit.

### Example 1

In order to improve the signal-to-noise ratio and complementary activity of the luciferase dual-antibody complementary activation assay, a series of complementary luciferase small subunits were prepared in this example. Large subunit-rSCFV5 fusion protein (SEQ ID NO: 87) was kept unchanged, and the small subunit sequence of the rSCFV18-small subunit P114 fusion protein (SEQ ID NO: 88) was modified by semi-randomization to prepare rSCFV18-small subunit mutant fusion proteins. Based on the sequence of small subunit P114, and with reference to the 3D structure of complementary luciferase, computer-aided sequence design was employed to generate a library with a theoretical diversity of 2.49*10^6. Primers were designed for PCR amplification using the plasmid pComb3x of rSCFV18-small subunit P114 fusion protein as a template. Subsequently, an expression vector library was constructed by means of restriction digestion and ligation (SfiI, NheI), and a semi-random amino acid mutation library was introduced into the small subunit position and transformed into HB2151 competent cells, yielding a library capacity of 7.6*10^9. The 100 plates were spread, and 9,500 single clones were picked and inoculated into 600 µL of 2YTA medium, followed by incubation at 37°C with shaking at 250 rpm for 2 h. When the bacterial density reached approximately OD₆₀₀ = 0.6~1.0, IPTG was then added to a final concentration of 1 mM, and the culture was further incubated at 30°C with shaking at 250 rpm for 20 h. The next day, the bacteria were collected by centrifugation at 4°C, 4,500g for 10min. The supernatant was discarded, and the precipitate was resuspended with 200µl of PBS containing polymyxin B at a final concentration of 20,000 u/ml. The suspension was incubated at room temperature with shaking at 100rpm for 1h, and then centrifuged at 4500g for 10min, and the supernatant was used for the test.

Test Methods:
(1) The concentration of large subunit-rSCFV5 fusion protein was diluted to 50 nM, and 15 µl was added per well;
(2) MBP-PCT fusion protein was diluted to 3.3 ng/ml with PBSAD, and 15 µl was added per well. The background control was PBSAD, and 10 µl was added per well;
(3) HB2151 strain containing expression plasmids with different small subunit were induced by 1 mM of IPTG at 30°C overnight, then treated with polymyxin B for 1.5 h, and centrifuged to collect the supernatant, and 2.5 µl supernatant was added per well;
(4) The above three components were slightly mixed, incubated at 37°C for 7 min, and Obuffer 6.37-3 containing Furimazine at a concentration of 25 µM was added, and after 80 s, the luminescence signal value was detected using a bioluminescence detector; and
(5) Clones with RLU higher than 10,000 and a signal-to-noise ratio (RLU antigen+/RLU antigen-) higher than that of the rSCFV18-small subunit P114 fusion protein were selected for retesting. The substrate was replaced with CTZ-h for retesting. Obuffer 6.37-3 containing CTZ-h at a concentration of 25 µM was added, and after 60 s, the luminescence signal value was detected using a bioluminescence detector.

The signal values, signal-to-noise ratios for PCT detection after complementation of the randomly mutated small subunit with the large subunit after retesting, and amino acid sequences of the small subunits are shown in Table 1 below.

**Table 1**

| **No.** | **Sequencing clone** | **PCT+** | **PCT-** | **Signal-to-noise ratio** | **Amino acid sequence** | **Sequence ID No.** |
|---|---|---|---|---|---|---|
| 1 | RB239 | 31936 | 465 | 67.7 | VEGYRLFDKKW | SEQ ID NO: 1 |
| 2 | RA141 | 30623 | 463 | 65.1 | VDGWRLHEKIS | SEQ ID NO: 2 |
| 3 | RB073 | 46672 | 953 | 48 | VEGYRLFDRKS | SEQ ID NO: 3 |
| 4 | RA116 | 55957 | 1204 | 45.5 | VEGWRLHEKIS | SEQ ID NO: 4 |
| 5 | RA128 | 17891 | 387 | 45.2 | VEGYRLHEKIS | SEQ ID NO: 5 |
| 6 | RB186 | 27190 | 590 | 45.1 | VEGYRLFDRKW | SEQ ID NO: 6 |
| 7 | RB135 | 35245 | 788 | 43.7 | VEGYRLFDRKL | SEQ ID NO: 7 |
| 8 | RB152 | 37150 | 906 | 40 | VEGYRLFKREL | SEQ ID NO: 8 |
| 9 | RB128 | 26168 | 658 | 38.8 | VEGYRLFDRVW | SEQ ID NO: 9 |
| 10 | RB085 | 47227 | 1206 | 38.2 | VEGYRLFEKVW | SEQ ID NO: 10 |
| 11 | RA120 | 81063 | 2139 | 36.9 | VSGWRIHEKIS | SEQ ID NO: 11 |
| 12 | RB172 | 41446 | 1094 | 36.9 | VEGYRLFEKVW | SEQ ID NO: 12 |
| 13 | RB240 | 50183 | 1384 | 35.3 | VEGYRLFERIW | SEQ ID NO: 13 |
| 14 | RB276 | 44654 | 1242 | 35 | VEGYRLFERKW | SEQ ID NO: 14 |
| 15 | RB223 | 39231 | 1099 | 34.7 | VEGYRLFNRKW | SEQ ID NO: 15 |
| 16 | RB209 | 37398 | 1080 | 33.6 | VEGYRLFDRKL | SEQ ID NO: 16 |
| 17 | RB212 | 33103 | 957 | 33.6 | VEGYRLFDRKL | SEQ ID NO: 17 |
| 18 | RB150 | 53945 | 1630 | 32.1 | VEGYRLFERVL | SEQ ID NO: 18 |
| 19 | RB035 | 58318 | 1790 | 31.6 | VEGYRLFERKW | SEQ ID NO: 19 |
| 20 | RB113 | 57092 | 1769 | 31.3 | VEGYRLFERIW | SEQ ID NO: 20 |
| 21 | RB195 | 41519 | 1307 | 30.8 | VEGYRLFNEKS | SEQ ID NO: 21 |
| 22 | RB075 | 76733 | 2735 | 27.1 | VEGYRLFNRKS | SEQ ID NO: 22 |
| 23 | RB033 | 109352 | 4101 | 25.7 | VEGYRLFERKS | SEQ ID NO: 23 |
| 24 | RB054 | 92160 | 3449 | 25.7 | VEGYRLFNKKS | SEQ ID NO: 24 |
| 25 | RB157 | 74319 | 2837 | 25.2 | VEGYRLFERKL | SEQ ID NO: 25 |
| 26 | RB027 | 101765 | 4316 | 22.6 | VEGYRLFNRVS | SEQ ID NO: 26 |
| 27 | RB097 | 95553 | 4346 | 21 | VEGYRLFERKS | SEQ ID NO: 27 |
| 28 | RB149 | 79060 | 3587 | 21 | VEGYRLFEKKL | SEQ ID NO: 28 |
| 29 | RB252 | 70963 | 3324 | 20.3 | VEGYRLFNRKS | SEQ ID NO: 29 |
| 30 | RB122 | 55355 | 2605 | 20.2 | VEGYRLFNRVG | SEQ ID NO: 30 |
| 31 | RB284 | 3993 | 302 | 12.2 | VEGYRLFNKEW | SEQ ID NO: 31 |
| 32 | P114 | 5058 | 414 | 12.2 | VTGYRLFEEIL | SEQ ID NO: 32 |

According to the results shown in Table 1: many small subunits, with comparable or even significantly higher signal-to-noise ratios and complementary activity than those of the rSCFV18-small subunit P114 fusion protein, were found by sequence modification of the small subunits.

### Example 2

Protein expression and purification of small subunit mutants:
The strains containing plasmids of different small subunits inTable 2, prepared in Example 1, were inoculated into 200ml of 2YTA medium and cultured at 37°C with shaking at 250 rpm for about 3h. When the OD600 reached 1.0, IPTG was added to a final concentration of 1mM, and the culture was further incubated at 30°C with shaking at 250rpm for 20h. The culture were centrifuged at 5,000g for 10min, and the precipitate was resuspended with 10ml of PBS, added with polymyxin B to 20,000u/ml. The suspension was inverted and mixed at room temperature for 1h, and centrifuged at 10,000g to collect the supernatant. The rSCFV18-small subunit mutant fusion protein was purified according to the standard procedure for his-tag protein purification using Ni-NTA resin.

### Test Methods:

In the same tube, the large subunit-rSCFV5 fusion protein was diluted with PBSA to 50 nM; rSCFV18-small subunit mutant fusion protein was diluted to 90 nM, mixed well, and then 22 µl was added per well; the MBP-PCT fusion protein was diluted in 3-fold gradient with PBSAD (in pg/ml), with 8 gradients in total, 10µl was added per well, and the background control was PBSAD and 10 µl was added per well; the plate was mixed with slight shaking and incubated at 37°C for 6 min, 50 µl of Obuffer 6.37-3 containing 25 µM of CTZ-h then was added, and after 20 s, the luminescence value was detected.

The signal-to-noise ratios for PCT detection of complementation activation of the large subunit-rSCFV5 fusion protein with the rSCFV18-small subunit mutant fusion protein are shown in Table 2 below.

**Table 2**

| | Small subunit | | | | | |
|---|---|---|---|---|---|---|
| PCT (pg/ml) | P114 | RA116 | RB073 | RB085 | RB135 | RB239 |
| 1000 | 1256 | 20492 | 16026 | 15446 | 15181 | 8952 |
| 333.3 | 656 | 7341 | 5886 | 5551 | 5480 | 3364 |
| 111.1 | 495 | 2700 | 2302 | 2102 | 2137 | 1318 |
| 37.04 | 435 | 1212 | 1013 | 932 | 943 | 590 |
| 12.35 | 400 | 602 | 591 | 556 | 575 | 407 |
| 4.12 | 383 | 473 | 475 | 445 | 422 | 332 |
| 1.37 | 398 | 406 | 358 | 384 | 367 | 301 |
| 0 | 333 | 362 | 412 | 337 | 380 | 288 |
| Signal-to-noise ratio at 1000 pg/ml | 1.44 | 56.6 | 38.9 | 45.8 | 40.0 | 31.1 |

According to the results shown in Table 2: the signal-to-noise ratios of the screened small subunit mutants were all relatively high. It is illustrated that the small subunits screened in the present application improve the sensitivity of the PCT detection by increasing the signal-to-noise ratio and reducing the background.

### Example 3

Test Methods:
(1) MBP-PCT protein was diluted with PBSAD in 10-fold gradient starting from 100 ng/ml, with 8 gradients in total, and 10 µl was added per well;
(2) The large subunit-SCFV5 fusion protein was diluted with PBSA to 100 nM, and 10 µl was added per well;
(3) Different rSCFV18-small subunit mutant fusion proteins were diluted with PBSA to 180 nM, and 10 µl of each was added per well;
(4) The plate was mixed slightly and incubated at 37°C for 10 min; and
(5) 50 µl of Obuffer 6.37-3 containing 25 µM of CTZ-h was added, and after 140 s, the luminescence value was detected. The results are shown after normalization against the background value.

The signal-to-noise ratios and linear ranges for PCT detection, comparing the small subunits obtained by the present invention with the control small subunits, are shown in Table 3 below. The standard curves of complementary activation of the rSCFV18-small subunit mutant fusion protein and the large subunit-SCFV5 fusion protein for PCT detection are shown in Figure 2.

**Table 3**

| Small subunit | Signal-to-noise ratio at 1000 pg/ml | 100000 | 10000 | 1000 | 100 | 10 | 3.3 | 1.1 | 0 |
|---|---|---|---|---|---|---|---|---|---|
| RB239 | 11.5 | 2167580 | 466004 | 57648 | 11004 | 599 1 | 5622 | 553 0 | 5000 |
| RA120 | 18.0 | 1999707 | 694625 | 90072 | 13450 | 586 0 | 5257 | 490 2 | 5000 |
| RB073 | 14.5 | 1966617 | 566678 | 72352 | 12151 | 590 2 | 5283 | 511 6 | 5000 |
| RB135 | 13.6 | 1936894 | 536667 | 67860 | 11916 | 573 1 | 5698 | 505 1 | 5000 |
| RB085 | 13.9 | 1830312 | 554514 | 69595 | 11950 | 575 4 | 5486 | 523 1 | 5000 |
| RA116 | 14.0 | 1784513 | 515360 | 69976 | 11673 | 563 2 | 5301 | 481 4 | 5000 |
| RA141 | 8.6 | 1399433 | 333241 | 42762 | 8685 | 602 5 | 5414 | 492 7 | 5000 |
| RB284 | 4.9 | 861878 | 187742 | 24677 | 7039 | 553 0 | 5288 | 509 2 | 5000 |
| P114 | 3.8 | 642399 | 129750 | 18839 | 7073 | 479 8 | 4789 | 498 1 | 5000 |

According to the results shown in Table 3 and Figure 2: the screened small subunits, such as RB239, RA120, RB073, RB085, RA116, RA141, and RB284, have higher signal-to-noise ratios in luciferase dual-antibody complementary activation for PCT detection, illustrating that signal-to-noise ratios reflect the subsequent sensitivity well.

### Example 4

In order to further improve the signal-to-noise ratio and complementary activity of the luciferase dual-antibody complementary activation assay, novel complementary luciferase small subunits were prepared in this example. Large subunit-rSCFV5 fusion protein (SEQ ID NO: 87) was kept unchanged, and the small subunit sequence of the rSCFV18-RB073 fusion protein (SEQ ID NO: 89) were modified by semi-randomization. The theoretical diversity was 6912, the library capacity was 10^6, and 3,000 single clones were picked. The method of protein induction and expression was the same as that in Example 2.

Detection Methods:
(1) The concentration of large subunit-rSCFV5 fusion protein was diluted to 12.5 nM, and 15 µl was added per well;
(2) MBP-PCT fusion protein was diluted to 3.3 ng/ml with PBSAD, and 10 µl was added per well;
(3) HB2151 strain containing plasmids with different small subunit sequences were induced by IPTG at 30°C overnight, then treated with polymyxin B for 1.5h, and centrifuged to collect the supernatant, and 2.5µl supernatant was added per well;
(4) The above three components were slightly mixed, incubated at 37°C for 7 min, and Obuffer 6.37-3 containing Furimazine at a concentration of 25 µM was added, and after 3 min, the luminescence signal value was detected using a bioluminescence detector; and
(5) Clones with RLU higher than 10,000 were picked. The substrate was replaced with CTZ-h substrate for retesting (Obuffer 6.37-3 containing CTZ-h at a concentration of 25 µM was added, and after 60 s, the luminescence signal value was detected using a bioluminescence detector), and a PBSAD control was added to test the signal-to-noise ratio.

The signal values, signal-to-noise ratios for PCT detection after complementation of the randomly mutated small subunit with the large subunit, and amino acid sequences of the small subunit are shown in Table 4 below.

**Table 4**

| No. | Sequencing clone | PCT- | PCT+ | Signal-to-noise ratio | Amino acid sequence | Sequence ID No. |
|---|---|---|---|---|---|---|
| 1 | RC-175 | 587 | 6953 | 11.8 | VDGWRLFDKKS | SEQ ID NO: 33 |
| 2 | RC-286 | 438 | 5003 | 11.4 | VEGYRIFEKKW | SEQ ID NO: 34 |
| 3 | RC-215 | 531 | 5972 | 11.2 | VEGYRLFDKVS | SEQ ID NO: 35 |
| 4 | RC-033 | 820 | 8509 | 10.4 | VSGYRLFDRKW | SEQ ID NO: 36 |
| 5 | RC-145 | 808 | 8198 | 10.1 | VEGYRLFERKS | SEQ ID NO: 37 |
| 6 | RC-042 | 593 | 5769 | 9.7 | VEGYRLFEKVS | SEQ ID NO: 38 |
| 7 | RC-178 | 593 | 5708 | 9.6 | VSGYRLFDKVS | SEQ ID NO: 39 |
| 8 | RC-224 | 1142 | 10561 | 9.2 | VDGWRLFDKKS | SEQ ID NO: 40 |
| 9 | RC-247 | 497 | 4398 | 8.8 | VEGYRLFDKVS | SEQ ID NO: 41 |
| 10 | RC-087 | 861 | 6923 | 8.0 | VEGYRLFDKVS | SEQ ID NO: 42 |
| 11 | RC-044 | 1302 | 10392 | 8.0 | VSGYRIFDKKS | SEQ ID NO: 43 |
| 12 | RC-187 | 1405 | 11049 | 7.9 | VSGYRLFDKKS | SEQ ID NO: 44 |
| 13 | RC-218 | 921 | 7242 | 7.9 | VEGYRLFDRKS | SEQ ID NO: 45 |
| 14 | RC-134 | 1223 | 9409 | 7.7 | VSGYRLFDKKS | SEQ ID NO: 46 |
| 15 | RC-171 | 1363 | 10309 | 7.6 | VDGWRLFERKS | SEQ ID NO: 47 |
| 16 | RC-124 | 1234 | 9280 | 7.5 | VSGWRIFDKKS | SEQ ID NO: 48 |
| 17 | RC-099 | 1164 | 8149 | 7.0 | VSGYRLFDKKW | SEQ ID NO: 49 |
| 18 | RC-263 | 685 | 4768 | 7.0 | VDGYRLFEKVS | SEQ ID NO: 50 |
| 19 | RC-018 | 1998 | 13188 | 6.6 | VSGYRLFDRKS | SEQ ID NO: 51 |
| 20 | RC-203 | 1536 | 10062 | 6.6 | VDGYRLFERKS | SEQ ID NO: 52 |
| 21 | RC-114 | 982 | 6401 | 6.5 | VSGYRLFDKVS | SEQ ID NO: 53 |
| 22 | RC-103 | 1337 | 8672 | 6.5 | VDGWRLFDKKS | SEQ ID NO: 54 |
| 23 | RC-208 | 2056 | 12303 | 6.0 | VSGYRLFDKVS | SEQ ID NO: 55 |
| 24 | RC-239 | 1676 | 10022 | 6.0 | VEGYRLFEKIS | SEQ ID NO: 56 |
| 25 | RC-242 | 1524 | 9037 | 5.9 | VNGWRIFDKKS | SEQ ID NO: 57 |
| 26 | RC-206 | 1260 | 7413 | 5.9 | VSGYRIFERKW | SEQ ID NO: 58 |
| 27 | RC-129 | 2068 | 12040 | 5.8 | VSGYRIFEKKS | SEQ ID NO: 59 |
| 28 | RC-082 | 2232 | 12852 | 5.8 | VEGYRLFEKIS | SEQ ID NO: 60 |
| 29 | RC-232 | 3183 | 18313 | 5.8 | VEGWRLFDRKS | SEQ ID NO: 61 |
| 30 | RC-220 | 1192 | 6738 | 5.7 | VSGYRLFDRVW | SEQ ID NO: 62 |
| 31 | RC-152 | 1715 | 9688 | 5.6 | VSGYRIFEKKS | SEQ ID NO: 63 |
| 32 | RC-036 | 1565 | 8563 | 5.5 | VEGYRLFEKIS | SEQ ID NO: 64 |
| 33 | RC-228 | 3000 | 16339 | 5.4 | VSGYRLFDRKS | SEQ ID NO: 65 |
| 34 | RC-113 | 1176 | 6207 | 5.3 | VSGYRIFEKKW | SEQ ID NO: 66 |
| 35 | RC-070 | 1715 | 8980 | 5.2 | VEGWRIFERKW | SEQ ID NO: 67 |
| 36 | RC-098 | 1698 | 8666 | 5.1 | VSGYRLFDRKS | SEQ ID NO: 68 |
| 37 | RC-271 | 1428 | 7132 | 5.0 | VNGWRLFDKKS | SEQ ID NO: 69 |
| 38 | RC-105 | 2934 | 14534 | 5.0 | VEGYRLFERKS | SEQ ID NO: 70 |
| 39 | RC-237 | 2186 | 10821 | 5.0 | VEGWRLFDRVS | SEQ ID NO: 71 |
| 40 | RC-032 | 1998 | 9519 | 4.8 | VSGYRLFDRVS | SEQ ID NO: 72 |
| 41 | RC-041 | 2495 | 11789 | 4.7 | VSGWRIFDRKS | SEQ ID NO: 73 |
| 42 | RC-261 | 1616 | 7217 | 4.5 | VSGYRLFDKKW | SEQ ID NO: 74 |
| 43 | P114 | 786 | 1493 | 1.9 | VTGYRLFEEIL | SEQ ID NO: 32 |

According to the results shown in Table 4, the signal-to-noise ratio of the complementary luciferase small subunit of this example is significantly higher than the signal-to-noise ratio of the small subunit P114.

### Example 5

In order to further improve the signal-to-noise ratio and complementary activity of the luciferase dual-antibody complementary activation assay, the preferred complementary luciferase small subunits obtained in Example 4 was comprehensively evaluated in this example, and their sensitivity and linear range for PCT detecton, when complemented with the large subunit-rSCFV5 fusion protein, were compared.

Detection Methods:
(1) The concentration of large subunit-rSCFV5 fusion protein was diluted with PBSA to 8.3~25nM, and 15µl was added per well;
(2) MBP-PCT fusion proteins were diluted with PBSAD in a downward gradient starting from 2.5 µg/ml, and 10 µl was added per well;
(3) The rSCFV18-small subunit mutant-containing fusion protein was diluted with PBSA to 30~180 nM, and 15 µl was added per well;
(4) The above three components were slightly mixed, incubated at 37°C for 15 min, and Obuffer 6.37-3 containing CTZ-h at a concentration of 15 µM was added, and the luminescence signal value was immediately detected using a bioluminescence detector (the luminescence value was normalized);

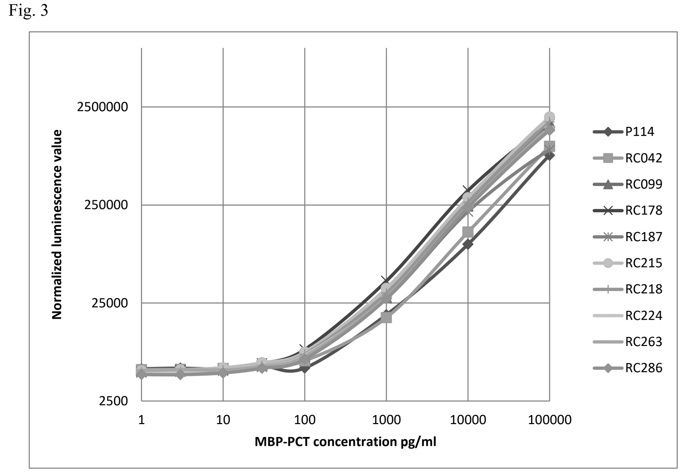

The signal-to-noise ratios and linear ranges for PCT detection, comparing the obtained small subunits with the control small subunits, are shown in Table 5 below. The standard curves of complementary activation of the rSCFV18-small subunit mutant fusion protein and the large subunit-SCFV5 fusion protein for PCT detection are shown in Figure 3.

**Table 5**

| Small subunit | Signal-to-noise ratio at 1000 pg/ml | 250000 | 100000 | 10000 | 1000 | 100 | 30 | 10 | 3 | 1 | 0.0 |
|---|---|---|---|---|---|---|---|---|---|---|---|
| P114 | 3.8 | 1431884 | 804042 | 99257 | 18939 | 5429 | 5724 | 5314 | 5403 | 5324 | 5000 |
| RC-099 | 5.7 | 2942238 | 1744023 | 243815 | 28688 | 7470 | 5950 | 5453 | 5166 | 5106 | 5000 |
| RC-178 | 8.4 | 1858964 | 1664322 | 350855 | 41922 | 8378 | 6047 | 5180 | 5152 | 4911 | 5000 |
| RC-187 | 5.5 | 1012760 | 910908 | 214329 | 27642 | 7032 | 5570 | 5099 | 4968 | 4944 | 5000 |
| RC-215 | 7.0 | 2983773 | 1967363 | 296125 | 35231 | 7892 | 6156 | 5458 | 5206 | 5095 | 5000 |
| RC-218 | 6.3 | 2436323 | 1757745 | 266213 | 31585 | 7494 | 5701 | 5164 | 4976 | 4937 | 5000 |
| RC-224 | 5.7 | 1856339 | 1453897 | 240337 | 28543 | 7341 | 5685 | 5260 | 5002 | 5021 | 5000 |
| RC-263 | 5.8 | 2230650 | 1556694 | 240937 | 28776 | 7134 | 5647 | 5192 | 5234 | 5132 | 5000 |
| RC-286 | 5.5 | 2007480 | 1443932 | 236023 | 27596 | 6696 | 5406 | 4833 | 4645 | 4670 | 5000 |

According to the results shown in Table 5 and Figure 3, the small subunit developed in the present invention has a significant advantage in terms of signal-to-noise ratio when applied to the luciferase dual-antibody complementation for PCT detection.

### Example 6

Through Example 1 and Example 4, it was found that the first position V, the third position G, and the fifth position R of the small subunit were highly conserved. In order to further determine whether these three conserved amino acid sequences were changed, this example constructed a random mutation library of these three positions, with a theoretical diversity of 8,000 and a library capacity of 10^6, and 2,000 single clones were picked. The method of protein induction and expression was the same as that in Example 1. The complementary activity was tested in the same manner as that in Example 4, but the signal-to-noise ratio was not retested with CTZ-h. Clones with different activity were randomly picked for sequencing.

The activity and sequence of the small subunit with random mutations at the conserved positions are shown in Table 6 below.

**Table 6**

| **Clone No.** | 3 min | 6 min | Small subunit sequence | Sequence number |
|---|---|---|---|---|
| **RD-012** | 3586 | 2963 | AEGYRLFDRKS | SEQ ID NO: 75 |
| **RD-045** | 2398 | 2087 | AEGYRLFDRKS | SEQ ID NO: 76 |
| **RD-084** | 2497 | 2394 | IEGYRLFDRKS | SEQ ID NO: 77 |
| **RD-086** | 3394 | 3222 | AEGYRLFDRKS | SEQ ID NO: 78 |
| **RD-092** | 4905 | 4539 | IEGYRLFDRKS | SEQ ID NO: 79 |
| **RD-098** | 214 | 49 | VEPYKLFDRKS | SEQ ID NO: 80 |
| **RD-099** | 221 | 52 | AEPYRIFDRKS | SEQ ID NO: 81 |
| **RD-100** | 44 | 214 | AESYKLFDRKS | SEQ ID NO: 82 |
| **RD-103** | 211 | 204 | VEGYHLFDRKS | SEQ ID NO: 83 |
| **RD-104** | 215 | 222 | VEPYKLFDRKS | SEQ ID NO: 84 |
| **RD-108** | 50 | 53 | AEGYHLFRSKS | SEQ ID NO: 85 |
| **RB073** | 46672 | 32973 | VEGYRLFDRKS | SEQ ID NO: 3 |

According to the results shown in Table 6, the vast majority of clones had no complementary activity or very low complementary activity. The first position V was changed to A or I with reduced but not complete loss of activity. However, the third position G, and the fifth position R were mutated to other amino acids with greatly reduced activity.

### Example 7

In order to verify whether the small subunit of the present invention still has good detection performance in other items, in this example, the target to be detected was replaced with human interleukin 6 (human IL6). The small subunit peptide RB073 was selected and fused with the human IL6 antibody for expression. The sensitivity and other detection performances of the reagents of this combination were evaluated.

Expression and purification of human IL6 recombinant protein: An expression vector of human IL6 (UniProtKB ID: P05231, AA29~212) with six histidine-tagged (C-terminal) fusion proteins (SEQ ID NO: 90) was constructed. The fusion protein was recombinantly expressed in E. coli, and purified with nickel column. The protein purity was detected by denaturing SDS-polyacrylamide gel electrophoresis, and the protein concentration was detected by nucleic acid-protein quantification instrument.

Expression and purification of a fusion protein of the small and large subunits with IL6 antibody: prokaryotic expression plasmids of the fusion protein RB073-449-1 (SEQ ID NO: 91) of the small subunit peptide RB073 with human IL6 SCFV antibody 449-1, and the fusion protein OL-453 (SEQ ID NO: 92) of the large subunit with human IL6 SCFV antibody 453 were constructed. The fusion proteins were recombinantly expressed in E. coli, and purified with nickel column. The protein purity was detected by denaturing SDS-polyacrylamide gel electrophoresis, and the protein concentration was detected by nucleic acid-protein quantification instrument.

Test Methods
(1) Recombinant human IL6 protein was diluted with PBSA in 10-fold gradient starting from 5000 pg/ml, with 5 gradients in total. 7 of zero-concentration wells were set, and 10 µl was added per well;
(2) Small subunit fusion protein RB073-449-1 was diluted with PBSA to 20 nM, and 15 µl was added per well;
(3) The large subunit fusion protein OL-453 was diluted with PBSA to 10 nM, and 15 µl was added per well;
(4) The plate was mixed slightly and incubated at 37°C for 5 min; and
(5) 50 µl of Obuffer 6.37-3 containing 50 µM of CTZ-h was added, and 10 s after addition of substrate, the RLU was immediately detected using a bioluminescence detector.

Data processing: LLD was estimated by ((mean+3SD)-RLU0.5)/(RLU5-RLU0.5)*4.5+0.5 pg/ml

**Table 7**

| IL6 pg/ml | RLU | IL6 pg/ml | RLU |
|---|---|---|---|
| 0 | 2291 | 0 | 2309 |
| 0 | 2344 | 0.5 | 2562 |
| 0 | 2259 | 5 | 2803 |
| 0 | 2287 | 50 | 5453 |
| 0 | 2484 | 500 | 33736 |
| 0 | 2261 | 5000 | 305924 |
| mean | 2321 | | |
| SD | 85.4 | mean+3 SD | 2577.4 |
| CV | 3.7% | LLD | 0.79pg/ml |

The results in Table 7 show that the small subunit peptide of the present invention, e.g., RB073, when used in the luciferase dual-antibody complementary activation assay for the detection of human IL6 calibrator has good sensitivity. The low limit of detection (LLD) is 0.79 pg/ml and a deviation in RLU of the zero-concentration is 3.7%. The sensitivity is expected to be not inferior to that of the products already on the market (e.g., the ADVIA Centaur IL6 from Siemens, USA, with a range of detection of 2.7-5500.0 pg/mL).

### Example 8

In order to validate the target detection performance of the small subunit of the present invention with the antibody by chemical coupling, a pair of antibodies to serum amyloid protein (SAA) were chemically coupled to the large and small subunits, respectively, in this example, and their detection performance was evaluated. Paired antibodies 011 and 941 were purchased from Shanghai Tianyu Biotechnology Co., Ltd. (Cat. No. 011, 941). Antibody 941 was coupled with a prokaryotic expression of OLC (SEQ ID NO: 105), and antibody 011 was coupled with the chemically synthesized polypeptide RB73.2 (SEQ ID NO: 106). The coupling method was performed according to the standard operating procedure of sulfo-SMCC (Thermo, 22322). The coupled antibody was obtained by purification of the antibody after coupling through molecular sieves or Protein G affinity purification.

Test Methods: The coupled antibodies 941-OLC and 011-RB73.2 were diluted with PBSA to 15 µg/mL, and 16.5 µL of each was added per well; the SAA antigen (Santa Scott, S-3SAA1) was gradient-diluted with PBSA to 0, 0.01, 0.1, 1, 10, and 100 mg/L, and 5 µL was added per well, slightly mixed and incubated at 37°C for 5 min; then, 50 µL of Obuffer 6.37-3 containing 50 µM of CTZ-h was added, and 10 s after substrate addition, RLU was immediately detected using a bioluminescence detector.

The results in Table 8 show that the coupled antibody pair of 941-OLC and 011-RB73.2 had excellent reactivity and linearity in SAA detection, with R²=0.9998 for linear correlation coefficient in the range of SAA concentration from 0.01 to 10 mg/L, which fully meets the requirements of clinical test reagents.

**Table 8**

| SAA concentration mg/L | RLU |
|---|---|
| 0 | 2324971 |
| 0.01 | 2435424 |
| 0.1 | 3875415 |
| 1 | 17038202 |
| 10 | 130656712 |

As can be seen from the above examples, the small subunit peptides of the present invention have good sensitivity and linear range in different detection items, and thus have a wide range of application prospects in the development of luciferase-based homogeneous immunodiagnostic reagents. These peptides have a very low background luminescence value in the absence of the target to be detected, and a very high luminescence efficiency in the presence of the antigen to be detected, when they form a luminescent complex in association with a large subunit fusion protein. Therefore, the small subunit peptides of the present invention, when used in luciferase dual-antidoby complementary activation assays, can reduce the background, improve the luminescence efficiency and the signal-to-noise ratio, and thereby improve the sensitivity of the detection.

The technical solution of the present invention is not limited to the limitations of the above specific examples, and all technical deformations made according to the technical solution of the present invention fall within the scope of protection of the present invention.

## Claims

1. A peptide comprising an amino acid sequence of 11 adjacent amino acids X₁X₂X₃X₄X₅X₆X₇X₈X₉X₁₀X₁₁, wherein,
X₁ is Val,
X₂ is selected from Glu, Ser, Asp, or Asn,
X₃ is selected from Gly,
X₄ is selected from Tyr or Trp,
X₅ is selected from Arg,
X₆ is selected from Leu or Ile,
X₇ is selected from Phe or His,
X₈ is selected from Asp, Glu, Asn, or Lys,
X₉ is selected from Lys, Arg, or Glu,
X₁₀ is selected from Lys, Val, Ile, or Glu, and
X₁₁ is selected from Ser, Trp, Leu, or Gly.

2. The peptide according to claim 1, wherein in the amino acid sequence,
X₁ is Val,
X₂ is selected from Glu, Ser, Asp, or Asn,
X₃ is selected from Gly,
X₄ is selected from Tyr or Trp,
X₅ is selected from Arg,
X₆ is selected from Leu or Ile,
X₇ is selected from Phe or His,
X₈ is selected from Asp, Glu, Asn, or Lys,
X₉ is selected from Lys, Arg, or Glu,
X₁₀ is selected from Lys, Val, or Glu, and
X₁₁ is selected from Ser, Trp, Leu, or Gly.

3. The peptide according to claim 1, wherein in the amino acid sequence,
X₁ is Val,
X₂ is selected from Glu or Asp,
X₃ is selected from Gly,
X₄ is selected from Tyr or Trp,
X₅ is selected from Arg,
X₆ is selected from Leu or Ile,
X₇ is selected from Phe or His,
X₈ is selected from Asp, Glu, Asn, or Lys,
X₉ is selected from Lys, Arg, or Glu,
X₁₀ is selected from Lys, Val, Ile, or Glu, and
X₁₁ is selected from Ser, Trp, or Leu.

4. The peptide according to claim 1, wherein when the peptide is contacted with a second peptide having an amino acid sequence with at least 70% sequence identity to SEQ ID NO: 86, a detectable luminescent signal is produced in the presence of a substrate, preferably the peptide is associated with the second peptide.

5. The peptide according to claim 1, wherein the peptide has an amino acid sequence selected from at least one of SEQ ID NOs: 1-31, and SEQ ID NOs: 33-74.

6. The peptide according to claim 1, wherein the peptide has a signal-to-noise ratio of ≥ 12, preferably ≥ 27, more preferably ≥ 30 for luciferase dual-antibody complementary target detection, when a concentration of the peptide is 30-360 nM, a concentration of the second peptide is 15-200 nM, and a concentration of the target to be detected is 1-120 pM; and/or
the peptide has a signal-to-noise ratio of ≥ 2, preferably ≥ 5, more preferably ≥ 7 for luciferase dual-antibody complementary target detection, when a concentration of the peptide is 0.2-30 nM, a concentration of the second peptide is 0.2-20 nM and a concentration of the target to be detected is 1-100 pM.

7. A nucleic acid molecule comprising a nucleotide sequence encoding the peptide according to any one of claims 1-6.

8. An expression vector comprising a nucleotide sequence of the nucleic acid molecule according to claim 7.

9. A host cell comprising the expression vector according to claim 8.

10. A conjugate comprising a first domain and a second domain, wherein
the first domain comprises the peptide according to any one of claims 1-6; and
the second domain comprises a first ligand.

11. The conjugate according to claim 10, wherein the first ligand is selected from an antigen or antigen-binding region,
preferably, the antigen-binding region comprises antibody, Fab fragment, Fab' fragment, F(ab')₂ fragment, Fv fragment, scFv fragment, nanobody, heavy chain variable region (VH) fragment or light chain variable region (VL) fragment.

12. The conjugate according to claim 10, wherein the first domain and the second domain are connected directly or via a first linker,
preferably, the first linker is selected from a polypeptide linker and/or a non-polypeptide linker, and preferably, the first linker is a flexible linker having a formula of (G)ₙS(G)ₘS(G)ₗS, wherein n is selected from 1 to 5, m is selected from 0 to 5, and l is selected from 0 to 5,
preferably, the first linker is a rigid linker having a formula of A(EAAAK)ₒA, wherein o is selected from 0 to 5,
more preferably, the first linker comprises GGGS, GGGSGGS (SEQ ID NO: 93), GGGSGGGGS (SEQ ID NO: 94), GGGSGGGSGGGS (SEQ ID NO: 95), or GGGGSGGGGSGGGGS (SEQ ID NO: 96),
more preferably, the first linker comprises AEAAAKA (SEQ ID NO: 97), AEAAAKEAAAKA (SEQ ID NO: 98), or AEAAAKEAAAKEAAAKA (SEQ ID NO: 99);
preferably, the first linker is a chemical linker,
more preferably, the first linker comprises one or more of acrylic phosphoramidite, adenylation, azide, digoxigenin, cholesterol-TEG, amino modifier, alkyne, biotinylation, thiol modification, tetraethylene glycol (TEG), hexaethylene glycol (HEG), polyethylene glycol (PEG), glycerol, C2 to C12 alkyl and succinimide.

13. A nucleic acid molecule comprising a nucleotide sequence encoding the conjugate according to any one of claims 10-12.

14. An expression vector comprising a nucleotide sequence of the nucleic acid molecule according to claim 13.

15. A host cell comprising the expression vector according to claim 14.

16. A luminescent complex comprising:
(a) the conjugate according to any one of claims 10-12; and
(b) the second conjugate,
wherein, the second conjugate comprises a third domain and a fourth domain, the third domain comprises a second peptide, and the fourth domain comprises a second ligand,
the second peptide is a structurally complementary peptide to the first peptide, and when the first peptide is associated with the second peptide, a detectable luminescent signal is produced in the presence of a substrate.

17. The luminescent complex according to claim 16, wherein the second ligand is selected from an antigen or antigen-binding region,
preferably, the antigen-binding region comprises antibody, Fab fragment, Fab' fragment, F(ab')₂ fragment, Fv fragment, scFv fragment, nanobody, heavy chain variable region (VH) fragment or light chain variable region (VL) fragment.

18. The luminescent complex according to claim 16, wherein the third domain and the fourth domain are connected directly or via a second linker;
preferably, the second linker is selected from a polypeptide linker and/or a non-polypeptide linker,
preferably, the second linker is a flexible linker peptide having a formula of GSS(G)ₚ(S)_{q}(G)ᵣ(S)ₛ(G)ₜ(S)ᵤ, wherein p is selected from 1 to 5, q is selected from 0 to 5, r is selected from 0 to 5, s is selected from 0 to 1, t is selected from 0 to 5, and u is selected from 0 to 5,
more preferably, the second linker comprises GSSG, GSSGGGS (SEQ ID NO: 100), GSSGGGSGGS (SEQ ID NO: 101), GSSGGGGSGGGS (SEQ ID NO: 102) or GSSGGGGSGGGGSGG (SEQ ID NO: 103);
preferably, the second linker is a chemical linker,
more preferably, the second linker comprises one or more of acrylic phosphoramidite, adenylation, azide, digoxigenin, cholesterol-TEG, amino modifier, alkyne, biotinylation, thiol modification, tetraethylene glycol (TEG), hexaethylene glycol (HEG), polyethylene glycol (PEG), glycerol, C2 to C12 alkyl and succinimide.

19. The luminescent complex according to claim 16, wherein the second peptide comprises an amino acid sequence with at least 70% sequence identity to SEQ ID NO: 86.

20. A luminescent complex comprising:
(a) a first peptide comprising the peptide according to any one of claims 1-5; and
(b) a second peptide being a structurally complementary peptide to the first peptide, wherein when the first peptide is associated with the second peptide, a detectable luminescent signal is produced in the presence of a substrate;
preferably, the second peptide comprises an amino acid sequence with at least 70% sequence identity to SEQ ID NO: 86.

21. A detection reagent comprising:
(a) a first peptide comprising the peptide according to any one of claims 1-6; and
(b) a substrate for a luminescent complex formed by association of the first peptide and a second peptide;
wherein the second peptide is a structurally complementary peptide to the first peptide, and when the first peptide is associated with the second peptide, a detectable luminescent signal is produced in the presence of a substrate;
preferably, the second peptide comprises an amino acid sequence with at least 70% sequence identity to SEQ ID NO: 86.

22. Use of the peptide according to any one of claims 1-6, the conjugate according to any one of claims 10-12, the nucleic acid according to claim 7 or 13, the expression vector according to claim 8 or 14, the host cell according to claim 9 or 15, the luminescent complex according to any one of claims 16-20, or the detection reagent according to claim 21 in a platform based on luciferase luminescence detection.
